# EUROPEAN PATENT SPECIFICATION

(11) **EP 0 352 922 B1**
(45) Date of publication and mention of the grant of the patent: **15.12.1993**
(21) Application number: 89306870.0
(22) Date of filing: 06.07.1989
(51) Int. Cl.: G01N 5/02, G01N 33/22, G01N 15/06

(54) **An improved test and apparatus for determining smoke emissivity of a fuel**
Verfahren und Vorrichtung zur Ermittlung der Rauchentwicklungseigenschaften eines Brennstoffes
Méthode de détermination de la capacité d'émission de fumée d'un combustible et appareil pour la mise en oeuvre de ladite méthode

(30) Priority: 26.07.1988 GB 8817733
(43) Date of publication of application: 31.01.1990
(73) Proprietor: Coal Industry (Patents) Limited, London SW1X 7AE (GB)
(72) Inventor: Beksa, John David, Chesterfield Derbyshire S42 5LJ (GB)
(74) Representative: Butler, Lance

(56) References cited:
- GB-A- 1 030 696
- GB-A- 1 246 805
- JOURNAL OF PHYSICS E; SCIENTIFIC INSTRUMENTS, vol. 7, no. 4, April 1974, pages 309-313; B. BILES et al.: "AC reflectrometry: a sensitive method of measuring smoke or reactive gas in air"

## Description

This invention concerns an improved test and apparatus for determining the smoke emissivity of a fuel.

In particular, but not exclusively, the invention has reference to a test for determining the smoke emissivity of a solid smokeless fuel.

A solid smokeless fuel for use on domestic open fires is defined in Hansard as one which emits less than 5g/hr of smoke when subjected to the test prescribed in BS 3841: 1972 "Method for the Measurement of Smoke from Manufactured Solid Fuels for Domestic Open Fires".

The Applicants produce a number of smokeless fuels which are tested on a regular basis in order to meet Department of the Environment requirements.

The existing test involves the combustion of a sample standard volumetric charge of fuel on an open fire, the amount of smoke being determined gravimetrically by collection in an electrostatic precipitator through which all the flue gases are passed. The test is carried out under specified conditions in which the average radiant output of the fire is adjusted to a level which is reasonably characteristic of normal domestic usage, the result being expressed as the mass of smoke produced per unit time.

The current test as described in the standard is of long duration, giving a feed back of at least three days which is quite unsuitable for quality control of the product. Indeed it is possible that the product can be sold before the test results are relayed to the plant.

The British Standard allows for the use of an alternative test for quality control purposes.

An object of the present invention is to provide an improved test and apparatus for determining the smoke emissivity of a fuel which will yield swifter and yet accurate results thereby enabling the application of tighter quality control.

According to one aspect of the invention a test for determining the smoke emissivity of a fuel comprises igniting the sample of fuel on the firegrate by means of the igniter element, burning the fuel for a set period of time in a flow of air provided by the induced and forced draught fans, passing the exhaust gases of combustion through a preweighed filter medium associated with the chimney and located downstream of the firegrate, terminating the combustion, and reweighing the filter medium to determine the smoke emissivity.

According to another aspect of the invention apparatus for determining the smoke emissivity of a fuel includes a firegrate incorporating an igniter element, a chimney disposed above and spaced from the firegrate, a filter medium associated with the chimney downstream of the grate, and an induced draught fan and a forced draft fan located respectively downstream and upstream of the filter medium and arranged to direct a flow of air to the firegrate and thence through the chimney and to and through the filter medium.

The firegrate may conveniently be made from stainless steel mesh, the igniter element providing a support therefor and a steel ring in use containing the firebed. The firegrate may be positioned over an air supply duct which may be formed of glass.

The filter medium is advantageously mounted on the top of the chimney and releasably held in that position, a 'Hartley' 3-piece funnel being located immediately downstream of the filter medium.

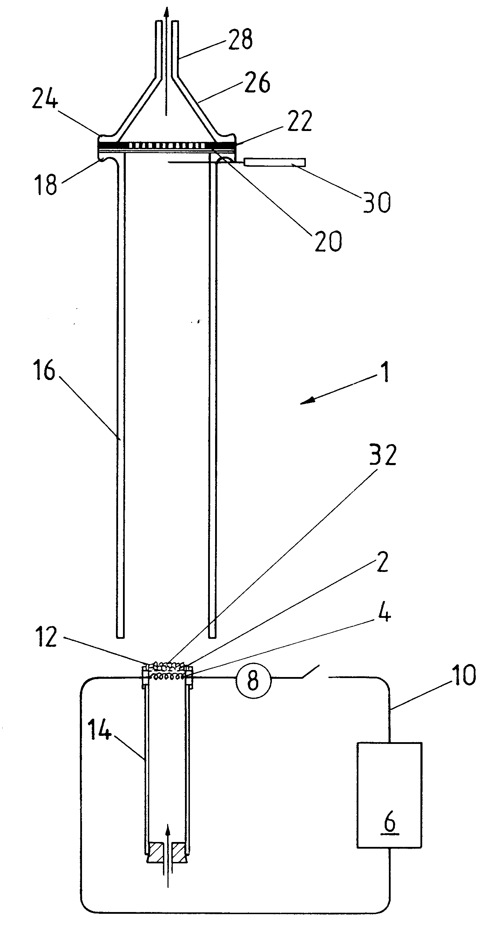

By way of example only, a test for determining the smoke emissivity of a fuel and one embodiment of apparatus therefor are described below with reference to the accompanying drawing which is a diagrammatic illustration of the apparatus.

Referring to the drawing, apparatus for determining the smoke emissivity of a fuel, in particular a solid smokeless fuel, such for example as that marketed under the Trade mark HOMEFIRE, is shown generally at 1 and comprises a firegrate 2 of stainless steel mesh surmounting and supported by an electrical igniter element 4 which draws its supply via a transformer 6, an ammeter 8 being included in the circuit 10. A steel ring 12 surrounds the firegrate 2 and the element 4 and is held on the top of a glass tube 14 through which in use combustion air is supplied from a forced draught fan (not shown).

A chimney 16 of stainless steel is superposed over the firegrate 2 and carries at its relatively upper end a flange 18 on which is positioned a filter medium in the form of two glass fibre filter discs 20 having a perforate support 22 thereabove, the filter discs 20 and the support 22 being releasably held in place by clips (not shown) between the flange 18 and another flange 24 on a Hartley funnel 26. The funnel 26 has an exhaust outlet 28 to which is connected tubing (not shown) leading to an induced draught fan (not shown). A temperature probe 30 extends into the chimney 16 just upstream of the filter discs 20.

On test that was carried out using the invention involved preparing a sample of the fuel referred to above by crushing to a size range of 1 to 3mm and then drying for a requisite period. The weight of the sample was elected to be 2g and the fuel was placed on the firegrate 2 and levelled to form a firebed 32 of even depth.

Two filter discs 20 were preweighed and placed at the top of the chimney 16 and clamped in position as previously described. The forced draught fan was started and set to give a delivery of 2 litre/min and the induced draught fan was set to give a pressure reduction of 100mm Hg above the filter medium.

The igniter element 4 was connected to the electrical supply for 2.5 minutes to give 200W of power to the firebed 32 in order to initiate combustion, after which the current was switched off to allow self-sustaining combustion to proceed for a further 2.5 minutes. The gases of combustion emanating from the firebed passed through the chimney 16 and the filters 20, the temperature at the lower of the two filters being approximately 60°C.

At the conclusion of the test run, namely at the expiration of 5 minutes, the two fans were switched off and the filter discs were removed and reweighed and the 'smoke' emissivity calculated as a percentage an a w/w basis.

It was found a series of test runs using the present invention yielded accurate results with a good correlation with the 'standard' test procedure as laid down in BS 3841 for a particular smokeless fuel. The present invention provides a rapid and repeatable method of determining the smoke emissivity of solid smokeless fuel and can thus be more readily used as a quality control.

## Claims

1. Apparatus for determining the smoke emissivity of a fuel characterised by a firegrate (2) incorporating an igniter element (4), a chimney (16) disposed above and spaced from the firegrate (2), a filter medium (20) associated with the chimney (16) downstream of the firegrate (2), and an induced draught fan and a forced draught fan located respectively downstream and upstream of the filter medium (20) and arranged to direct a flow of air to the firegrate (2) and thence through the chimney (16) and to and through the filter medium (20).

2. Apparatus according to Claim 1 characterised in that the firegrate (2) is formed of a stainless steel mesh.

3. Apparatus according to Claim 1 or 2 characterised in that the igniter element (4) provides a support for the firegrate (2).

4. Apparatus according to any one of the preceding claims characterised in that a ring (12) surrounds the firegrate (2) and the igniter (4) for containing a firebed (32) of the fuel to be tested.

5. Apparatus according to any one of the preceding claims characterised in that the filter medium (20) is mounted on the top of the chimney (16) and is releasably held in that position.

6. Apparatus according to any one of the preceding claims characterised in that the filter medium comprises at least one filter disc (20).

7. A test of determining the smoke emissivity of a fuel using the apparatus according to any one of Claims 1 to 6 characterised by the steps of igniting the sample of fuel (32) on the firegrate (2) by means of the igniter element (4), burning the fuel for a set period of time in a flow of air provided by the induced and forced draught fans, passing the exhaust gases of combustion through a preweighed filter medium (20) associated with the chimney (16) and located downstream of the firegrate (2), terminating the combustion, and reweighing the filter medium (20) to determine the smoke emission.

## Patentansprüche

1. Vorrichtung zur Ermittlung der Rauchentwicklungseigenschaften eines Brennstoffes, gekennzeichnet durch einen Rost (2), welcher ein Zündelement (4) enthält, einen Abzug (16), welcher oberhalb und im Abstand von dem Rost (2) angeordnet ist, ein Filtermedium (20) mit dem Abzug (16) stromabwärts des Rostes (2) verbunden und ein Saugzuggebläse und ein Zwanszuggebläse, welche stromabwärts bzw. stromaufwärts des Filtermediums (20) angeordnet und derart ausgebildet sind, dass ein Luftstrom zu dem Rost (2), anschliessend durch den Abzug (16) und zu und durch das Filtermedium (20) erzeugt wird.

2. Vorrichtung nach Anspruch 1, dadurch gekennzeichnet, dass der Rost (2) aus einem rostfreien Stahlsieb besteht.

3. Vorrichtung nach Anspruch 1 oder 2, dadurch gekennzeichnet, dass das Zündelement (4) die Lagerung für den Rost (2) bildet.

4. Vorrichtung nach einem der vorstehenden Ansprüche, dadurch gekennzeichnet, dass ein Ring (12) den Rost (2) und das Zündelement (4) umgibt, um ein Feuerbett (32) aus dem zu untersuchenden Brennstoff aufzunehmen.

5. Vorrichtung nach einem der vorstehenden Ansprüche, dadurch gekennzeichnet, dass das Filtermedium (20) auf der Oberseite des Abzugs (16) montiert und in dieser Stellung lösbar gehalten ist.

6. Vorrichtung nach einem der vorstehenden Ansprüche, dadurch gekennzeichnet, dass das Filtermedium aus mindestens einer Filterscheibe (20) besteht.

7. Verfahren zur Ermittlung der Rauchentwicklungseigenschaften eines Brennstoffs unter Verwendung der Vorrichtung nach einem der Ansprüche 1 bis 6, gekennzeichnet durch folgende Verfahrensschritte:
Entzünden der Probe des Brennstoffs (32) auf dem Rost (2) mittels des Zündelements (4), Verbrennen des Brennstoffs über eine feste Zeitspanne in einer Luftströmung, welche durch das Saugzuggebläse und das Zwangszuggebläse erzeugt wird, Leiten der Abgase der Verbrennung durch ein zuvor gewogenes Filtermedium (20), welches mit dem Abzug (16) verbunden und stromabwärts des Rostes (2) angeordnet ist, Beenden der Verbrennung und erneues Wiegen des Filtermediums (20) um die Rauchentwicklung zu bestimmen.

## Revendications

1. Appareil de détermination du pouvoir émissif de fumées d'un combustible, caractérisé par une grille (2) comprenant un élément allumeur (4), une cheminée (16) placée au-dessus de la grille (2) et à distance de celle-ci, un milieu de filtration (20) associé à la cheminée (16) et placé en aval de la grille (2), et un ventilateur aspirant et un ventilateur soufflant placés respectivement en aval et en amont du milieu de filtration (20) et destinés à diriger un courant d'air vers la grille (2) puis dans la cheminée (16) et vers le milieu de filtration (20) et à travers celui-ci.

2. Appareil selon la revendication 1, caractérisé en ce que la grille (2) est formée d'une toile d'acier inoxydable.

3. Appareil selon la revendication 1 ou 2, caractérisé en ce que l'élément allumeur (4) forme un support pour la grille (2).

4. Appareil selon l'une quelconque des revendications précédentes, caractérisé en ce qu'un anneau (12) entoure la grille (2) et l'allumeur (4) afin qu'il contienne un lit de combustion (32) formé du combustible qui doit être testé.

5. Appareil selon l'une quelconque des revendications précédentes, caractérisé en ce que le milieu de filtration (20) est monté à la partie supérieure de la cheminée (16) et est maintenu temporairement dans cette position.

6. Appareil selon l'une quelconque des revendications précédentes, caractérisé en ce que le milieu de filtration comporte au moins un disque (20) de filtration.

7. Test de détermination du pouvoir émissif de fumées d'un combustible, à l'aide de l'appareil selon l'une quelconque des revendications 1 à 6, caractérisé par des étapes d'allumage de l'échantillon de combustible (32) sur la grille (2) à l'aide de l'élément allumeur (4), de combustion du combustible pendant une période réglée dans un courant d'air formé par les ventilateurs aspirant et soufflant, de circulation des gaz d'échappement de la combustion à travers un milieu (20) de filtration préalablement pesé, associé à la cheminée (16) et placé en aval de la grille (2), de terminaison de la combustion, puis de nouvelle pesée du milieu de filtration (20) pour la détermination de l'émission des fumées.
